(19) Europäisches Patentamt
European Patent Office
Office européen des brevets

(11) **EP 2 362 803 B1**

(12) **EUROPEAN PATENT SPECIFICATION**

(45) Date of publication and mention
of the grant of the patent:
**04.03.2015 Bulletin 2015/10**

(51) Int Cl.:
***A63B 69/36*** *(2006.01)*     ***A61B 5/103*** *(2006.01)*

(21) Application number: **09796022.3**

(22) Date of filing: **06.11.2009**

(86) International application number:
**PCT/GB2009/002620**

(87) International publication number:
**WO 2010/052468 (14.05.2010 Gazette 2010/19)**

(54) **MOTION ANALYSIS APPARATUS**

BEWEGUNGSANALYSEGERÄT

APPAREIL D'ANALYSE DE MOUVEMENT

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **07.11.2008 GB 0820473**

(43) Date of publication of application:
**07.09.2011 Bulletin 2011/36**

(73) Proprietor: **Initial Force AS**
**7491 Trondheim (NO)**

(72) Inventors:
• **LARSEN, Andreas**
**7560 Vikhammer (NO)**
• **KROG, Øystein, Eklund**
**7047 Trondheim (NO)**
• **RATHE, Kristian**
**7051 Trondheim (NO)**
• **MYKLEBUST, Thor**
**7562 Hundhamaren (NO)**
• **ETTEMA, Gerardus, Johannes, Cornelis**
**7054 Ranheim (NO)**
• **AUNE, Aage**
**7043 Trondheim (NO)**
• **BRAATEN, Steinar**
**7056 Ranheim (NO)**
• **STØREN, Ole**
**7052 Trondheim (NO)**

(74) Representative: **Magrath, Sally Ann Mason**
**Dehns**
**St Bride's House**
**10 Salisbury Square**
**London EC4Y 8JD (GB)**

(56) References cited:
**WO-A1-2006/120658     DE-A1-102006 005 558**
**FR-A1- 2 896 401     US-A1- 2006 194 178**
**US-B1- 6 402 635**

EP 2 362 803 B1

**Description**

The present invention relates to an apparatus for use in analysing the motion of a subject, in particular a force plate upon which a person stands. The apparatus has particular application in analysing human motion in sports, such as golf and baseball.

There are many situations in which the analysis of the motion of a person is desirable, for example in medicine for analysing the movement of a patient, and in a variety of training scenarios for providing feedback and instruction. One area in which motion analysis is used a great deal is in sports training and practice. In sports such as golf, the result of the shot depends considerably on the position and movement of the player before and during the swing. It is therefore not surprising that a number of systems have been developed that provide data relating to the movement of a player and/or provide analysis and feedback on that player's movement in relation to the swing. Many such systems work in conjunction with a golfing simulator for indoor practice.

Some known systems such as that disclosed in US 2007/0196800 A1 dynamically capture information concerning a golfer's body position and movement through a high resolution pressure mat under the golfer's feet. The pressure mat comprises an array of pressure sensors, and by analysing the pressure measurements from these sensors a number of features can be calculated such as the position of each foot, stance width, centre of gravity and weight distribution between the feet.

Another system disclosed in WO 2006/120658 uses two foot platforms, one for each foot, on which the player stands. Each foot platform has an arrangement of force sensors such as strain gauges, to measure the vertical and horizontal forces exerted by the player over time. A computer calculates various quantities such as the relative weight on each foot, weight transfer etc, from which characteristics such as pelvic rotation can be estimated. Two foot platforms are used so that the forces due to each foot can be distinguished. Without this information, quantities like relative weight could not be found.

The present inventors have recognised that such prior art systems have a number of disadvantages. The above system utilising a pressure mat can only provide data based on pressure measurements, and so for example the evaluation of horizontal and rotational motion, both of which are very important in the analysis of a golf swing, is not possible. Furthermore, even vertical motion is estimated indirectly from the pressure mat measurements, since there is no direct measurement of force. This introduces inaccuracies.

On the other hand, whilst direct forces can be measured using a system comprising two foot platforms, the player is not free to stand in a natural way, since he must place one foot on each platform in a certain way so that the measured forces are correctly attributed.

The present invention refers to a sports motion analysis apparatus as defined in claim 1, a sports motion analysis system as defined in claim 9 and a method for analysing the motion of a human subject in sports as defined in claims 11 and 12.

According to a first aspect, the present invention provides a sports motion analysis apparatus for use in analysing the motion of a human subject in sports, comprising a single platform upon which the subject stands, the platform comprising force sensors arranged to measure force exerted on the platform by the subject in each of three dimensions; the apparatus further comprising a foot position data collector arranged to collect data relating to the position of each foot of the subject on the platform; and an output arranged to output the force data and data relating to the position of each foot.

The platform is preferably in the form of a force plate and is sometimes referred to hereinafter as such.

The terms "motion of a human subject", "motion information" and "motion characteristics" used herein include not only vector quantities that intrinsically involve a time component and thus "motion", such as torque and rotational forces, but also those quantities which will change as the subject moves such as weight distribution and centre of pressure.

It will be appreciated that the meaning of a subject "standing" on a platform includes the subject moving about whilst situated on the platform, e.g. by leaning to one side, applying a torque through the feet etc. Furthermore the forces "exerted" by the subject encompasses all forces such as forces due to gravity, torque specifically applied by the subject etc.

Measuring force in each of three dimensions means for example measuring force in each of three perpendicular directions, commonly two perpendicular directions in an approximately horizontal plane, and a third direction perpendicular to the first two directions, in an approximately vertical plane.

To be able to calculate certain types of motion information, both measurements of the forces exerted by the subject and the position of the feet are required. Thus, since the force plate according to the invention collects data relating to both of these, it enables such types of motion information to be accurately calculated. Changes in balance and weight distribution over time, and body rotation, are types of motion information that require both force and foot position measurements, and thus the present invention enables them to be calculated.

Preferably, the data relating to the position of each foot is used in conjunction with data relating to the typical shape of a foot to determine the orientation of each foot (i.e. which direction each is facing). Such information is useful in calculating motion information.

The foot position data collector can take any suitable form. For example it may comprise visual/infrared markers on the feet and an associated camera. It may comprise capacitive proximity sensors. It may alternatively comprise an optical grid located just above the force plate surface, wherein each line in the grid detects whether a foot is blocking it. Technology used in touch-sensitive monitors may be used.

In one particularly preferred embodiment the foot position data collecting means comprises a pressure sensitive mat. Any suitable type of pressure mat may be used which collects pressure data relating to the position of the feet. In one preferred embodiment the pressure mat comprises a grid of force sensitive resistors (FSRs). Force sensitive resistors change resistivity when pressed down and are commercially available. In one embodiment, the mat comprises 1000 FSRs on a mat of 800mm x 500mm, resulting in a sensor spacing of 20mm. In another embodiment, a higher resolution is provided using a mat comprising 2048 FSRs resulting in a sensor spacing of approximately 14 to 15 mm. In a further embodiment the pressure mat comprises piezoelectric sensors which generate voltage when pressure is exerted on them.

In one embodiment it is necessary to determine only the outline position of the feet and not the amount of pressure exerted by the feet. Thus, the mat can be inexpensive and easy to implement compared with pressure mats used in the prior art because less sensors are needed and less processing is required.

In an alternative embodiment, data collected by the pressure mat can be used to find the pressure distribution for each foot, which can be used to evaluate the balance of the subject. For example, it can be used to determine if the subject is standing flat on his feet or if any part of the foot (heel, toes, left/right side) is partially or fully lifted from the ground. This information can be visualised on a screen.

A conventional three dimensional cartesian co-ordinate system is used in this application, comprising three mutually perpendicular X, Y and Z axes. In the following it is generally considered that the X and Y axes are perpendicular to each other in the horizontal plane, and the Z axis is perpendicular to the X and Y axes in the vertical plane.

The force sensors are arranged to measure force in three dimensions. Preferably, for optimal measurements, force sensors are provided that align with each axis/direction. For example at least one X force sensor may be aligned with the X axis, at least one Y force sensor may be aligned with the Y axis, and at least one Z force sensor may be aligned with the Z axis. Alternatively, force sensors may not be aligned with the axes, but may simply be arranged to measure forces from which X, Y and Z components can be calculated.

The combination of three dimensional force information in particular, together with foot position data, provides considerable new possibilities in the analysis of human motion. For example, features relating to body rotation such as torque, momentum and rotational strength can be evaluated in all three dimensions which is simply not possible with prior art systems.

In the prior art as discussed above, two force plates (one for each foot) are needed in order to distinguish between the forces of each foot. In contrast, in the present invention, a single foot platform / force plate can be used for both feet, since a pressure sensitive mat is used to collect data relating to the foot positions. This pressure data enables the forces to be correctly attributed to each foot. Using only a single foot platform / force plate simplifies manufacture and allows the subject to stand naturally on the force plate rather than having to place one foot on each force plate in a contrived manner as necessary in the prior art. Importantly, using only one force plate reduces cost since only one set of sensors is required to analyse both feet.

The apparatus of the present invention also enables motion to be analysed much more accurately due to measurement of both force and pressure data.

In one particularly preferred embodiment the apparatus is arranged for use in analysing the motion of a subject during a golf swing, though it has a wider application both in sports (for example in analysing motion during a baseball swing).

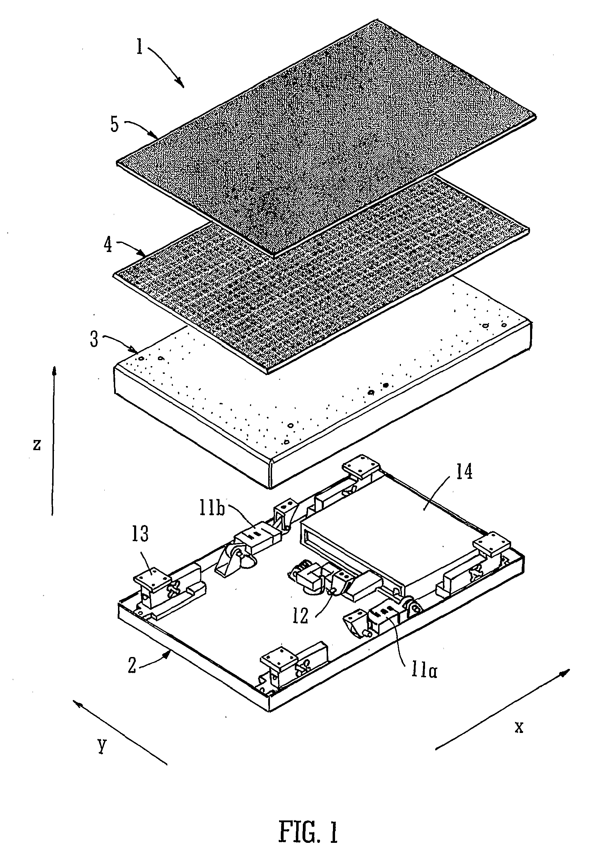

The force sensors can be any suitable sensors that measure force such as strain gauge load transducers. As discussed above, to enable a wide range of motion information to be calculated, sensors are provided to measure forces in the X, Y and Z directions. Preferably the platform / force plate is rectangular. If it is considered to have a front and a back, and the front is taken as the side to which the toes are directed, the back being taken to be the opposite side (i. e. the side to which the heels are directed) the X direction is considered here as the direction perpendicular to the direction the feet face when placed on the force plate (this is shown in the example of Figure 1). The Y direction is considered here as the direction parallel to the direction the feet face and the Z direction is the vertical direction (again shown in Figure 1).

In a preferred embodiment, two X sensors are provided (although in other embodiments more or less are provided), which are strategically placed in order to enable the most accurate measurements of horizontal forces in the X direction.

Preferably one X sensor is placed towards the front and one towards the back of the force plate. Preferably both sensors are centred halfway along the force plate in the X direction. As will be seen in more detail below, the X sensor data is particularly useful in analysing body rotation around the Z axis. The torque exerted on the force plate by the subject turning themselves clockwise/anticlockwise while the feet remain stationary, i.e. the torque around the Z axis, is equal to the cross product of force exerted by the feet around the Z axis and distance from the point about which the force causes rotation, i.e. the centre of rotation of the feet. Since the force is measured at the position of the X sensors, each X sensor should be located at approximately the same distance from the centre of rotation as it can be expected the average subject's feet will be placed. Thus, each X sensor should be placed along the Y axis at approximately the same distance from the centre of rotation as each foot, in order for torque to be accurately estimated using the X sensors.

Although the centre of rotation will vary depending on the position of the feet, since the X sensors will generally need to be fixed in place on the force plate prior to use, it is necessary for this purpose to pre-estimate the centre of rotation. Here, the centre of rotation is assumed to be approximately the force plate centre, thus the distance along the Y axis from the force plate centre to each X sensor should be approximately equal to the distance from the centre of the force plate to each foot. This enables as close to a 1:1 mapping as possible between measured forces and the actual measured forces when evaluating rotation around the Z axis, described later. The phrase "spin kick" is sometimes used in this application to refer to Z-axis torque, particular when this is discussed in a golfing context. This "spin kick" describes the force travelling in a circular path, with a diameter equal to the stance width. If the subject stands perfectly centred on the force plate, this "circle of force" passes approximately straight through both X-sensors parallel to their measuring direction, i.e. the x-axis. Therefore preferably the subject stands centred on the force plate.

Thus it can be understood that in a preferred embodiment, the X sensors are arranged towards the front and back of the force place respectively at roughly the same distance from the centre as which the feet are located on each side. In the case of use of the force plate in analysing golf swings, the width of an average golf stance (distance between feet when hitting a golf ball) is about 50cm, thus the distance from each foot to the centre of rotation is approximately 25cm. Each X sensor is therefore positioned in the Y direction approximately 25cm from the force plate centre. This enables the "spin kick" to be measured more accurately.

In one preferred embodiment one Y sensor to measure forces in the Y direction is provided towards the centre of the force plate; most preferably in the centre. In an alternative embodiment two or more Y sensors are provided. For example two Y sensors could be provided towards the left and right sides of the force plate in the same manner as the X sensors are provided towards the front and back. Thus preferably, each of two Y sensors would be arranged halfway along the force plate in the Y direction and approximately 25cm in the X direction from the force plate centre.

In one preferred embodiment four Z sensors are provided to measure forces in the vertical Z direction. These force sensors are preferably spaced out around the force plate, in the case of a rectangular force plate they are most preferably located in each of the four corners.

The force plate preferably includes a base plate on which the force sensor(s) are mounted, a cover plate disposed over the base plate and the pressure mat disposed over the cover plate. Preferably the cover plate is arranged to move freely on the force sensors, such that its movement is only restricted by the sensors and thus the forces absorbed by the mounting structure are minimised. In the case of golf swing analysis, preferably an artificial turf mat is arranged on top of the pressure mat for the subject to stand on. This provides the subject with a more natural environment. Preferably, the force plate further comprises electronics arranged to control the force sensors and pressure mat, collect data from them and output this data. This data is then preferably input to a processing means, for example a computer, which is arranged to process the data and calculate information relating to the motion of the subject.

Accordingly, the invention also provides a sports motion analysis system for analysing the motion of a human subject in sports, comprising an apparatus according to the first aspect of the invention as described above, a processing means arranged to determine some information relating to the motion of the subject using the measured forces in three dimensions and/or data relating to the position of each foot, and an output to output the motion information. Preferably the information is determined using both the measured forces and foot position data.

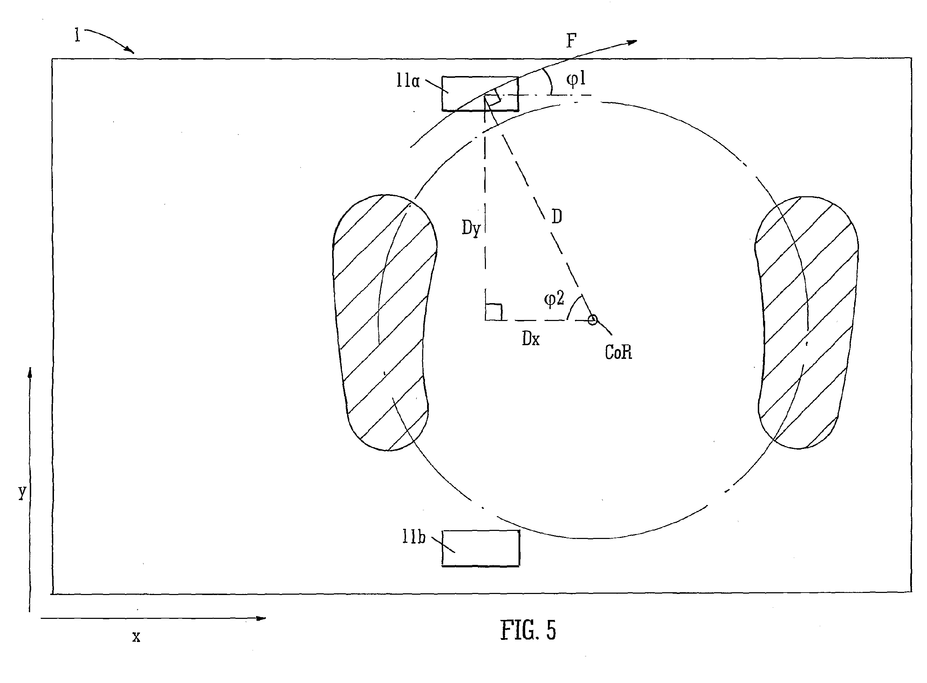

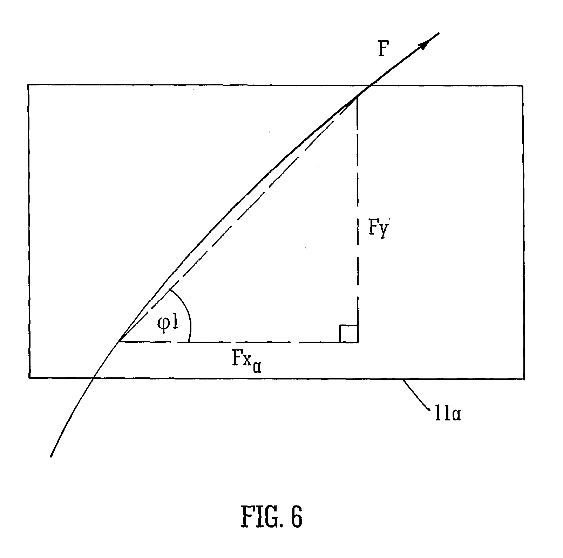

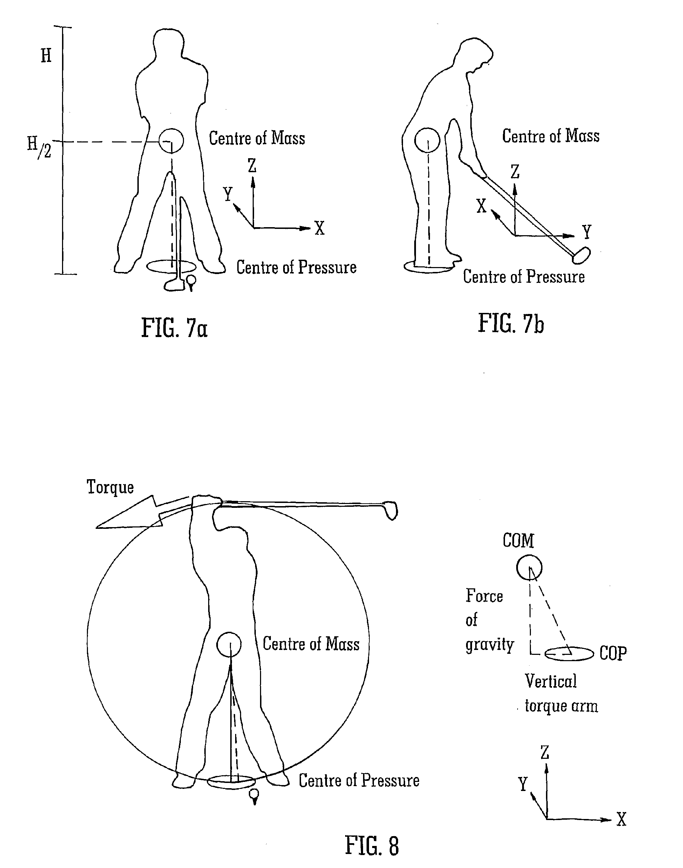

Generally, when the foot position data collecting means comprises a pressure sensitive mat, the processing means is arranged to estimate the outline of each foot on the force plate from the pressure mat data and use this together the measured force in three dimensions to calculate relevant motion information such as rotational force of the subject or balance. Details of types of motion information which are particularly useful to analyse in a golfing context are described below in Tables 1 and 2. The torque exerted by the feet around the Z axis (denoted elsewhere in a golfing context as "spin kick") can be found using the X sensor force measurements and the estimated foot positions according to the method as described later in relation to Figures 5 and 6. The body rotation around the Y and X axes can be found using the X and Y sensor data respectively together with the vertical Z sensor data and the position of the feet. For this rotation the body is seen as a sphere with a diameter equal to the subject's height. A preferred method is described later in relation to Figures 7-10.

The processing means may be external to the force plate, integral with it, or partly external and partly integrated. For example, the force plate may include electronics arranged to estimate the outline of each foot from the pressure mat data and to output this together with the force measurements to an external processing means which then performs further calculations.

The processing means preferably comprises a computer having software which when executed by the computer causes the computer to determine the motion information. Preferably the software includes a user interface, most preferably a graphical user interface, by which a person can interact with the system. Preferably the output is a monitor which displays the calculated motion information, which in one preferred embodiment is a touch-screen monitor.

The system may include further components such as a video camera arranged to record video of the movement of the subject, which is then displayed along with calculated motion information on the monitor. More than one video camera may be provided in order to take video at different angles, in one preferred embodiment four video cameras are provided.

In one embodiment, the system is arranged to analyse the motion of a human subject during a golf swing. In this context the system has particular application as a golf training and practice tool. The software is preferably arranged to provide analysis of and feedback on the measured motion characteristics. The system may be used as a practice tool by a student alone (whose motion is analysed), or with an instructor.

In this context, other components may be included for providing information relevant to a golf swing, such as a sensor capable of measuring aspects of the motion of the golf club. Suitable sensors include doppler radar sensors, ultrasound positioning sensors, laser technology and high speed cameras. A sensor may also be included which is capable of measuring aspects of the ball motion such as ball speed and vertical launch angle. Suitable sensors include doppler radar, laser technology and highspeed cameras. In a preferred embodiment a TrackMan™ (http://trackmangolf. com/) launch monitor is used which evaluates the motion of the golf club and ball using doppler radar.

Sensors for measuring specific aspects of the body motion may also be included, for example a motion-tracking suit or motion capture device.

A trigger sensor may also be included which reacts to a specific event in the motion of the subject, such as the striking of the ball. For example, a microphone may be included and obtained sound information used to determine the strike of the ball from the distinct sound it makes. Alternatively, any ball or club motion sensor may be used, for example one utilising doppler radar.

The force plate may be integrated into a tee unit which tees the ball. A commercially available tee unit may be used, or alternatively a bespoke tee unit may be provided. Preferably the force plate is integrated into the tee unit so that the turf mat of the force plate is flush with the turf of the tee unit.

It will be understood that whilst these additional components are described in the context of golf swings, they may also be applicable in other sports and activities.

In a broader aspect, the present invention provides a sports motion analysis system for analysing the motion of a human subject in sports, comprising a single platform upon which the subject stands, the platform comprising force sensors arranged to measure force exerted by the subject in each of three dimensions; the system further comprising a foot position data collector arranged to collect data relating to the position of each foot of the subject; and a processor arranged to determine information relating to the motion of the subject using measured forces and/or position data of each foot. Preferably the motion information is determined using both force and foot position data. The foot position data collecting means can be any suitable means for collecting data relating to the individual foot positions including orientation and outline. However in a preferred embodiment the foot position data collecting means is a pressure mat as described previously. Most preferably the foot position data collecting means is a pressure mat and is integrated with the force sensor into a force plate as previously described.

The system may be a golf practice system, wherein the platform is one on which the subject stands whilst practising a golf swing, wherein the processor is arranged to receive the force data and data relating to the position of each foot, determine the position of each foot of the subject and evaluate motion characteristics of the subject from the force data and foot position data; the system further comprising a display arranged to display the calculated motion characteristics; wherein preferably the platform is integrated into a tee unit.

The invention also extends to a method of analysing the motion of the human subject comprising the use of any of the above described apparatuses or systems.

In a further aspect the invention provides a method of analysing the motion of a human subject in sports, comprising: measuring force exerted on a single platform in each of three dimensions by a subject standing on the platform; collecting data relating to the position of each foot of the subject on the platform; and outputting the measured force data and data relating to the position of each foot.

In yet another aspect the invention provides a method of analysing the motion of a human subject in sports, comprising: measuring force exerted on a single platform in each of three dimensions by the subject standing on the platform; collecting data relating to the position of each foot of the subject on the platform; evaluating some information relating to the motion of the subject using both the measured forces and data relating to the position of each foot; and outputting the motion information.

In a further aspect, the invention provides a data processing apparatus configured to carry out the steps of the method of claim 11, 12 or 13.

In another aspect the invention provides a computer program product carrying instructions which when carried out by a data processing apparatus will configure the data processing apparatus to be in accordance with the above described data processing apparatus.

The computer program product can be in the form of a physical carrier bearing software, or in the form of signals transmitted from a remote location. The computer program product may also be described as a software product.

The invention also provides a method of manufacturing a software product which is in the form of a physical data carrier, comprising storing on the data carrier instructions which when carried out by a data processing apparatus will configure the data processing apparatus to be in accordance with the above-described data processing apparatus.

The invention also provides a method of providing a computer program product to a remote location by means of transmitting data to a data processing apparatus at that remote location, the data comprising instructions which when carried out by the data processing apparatus will configure the data processing apparatus to be in accordance with the above described data processing apparatus.

The data processing apparatus can be a computer.

Various features described previously and subsequently are applicable to such software products, for example in one preferred embodiment the information relating to the motion of the subject is torque or angular momentum around the X, Y and/or Z axes.

It will be understood that preferred features described in relation to certain aspects and embodiments may also be applicable to other aspects and embodiments and vice versa.

Preferred embodiments of the present invention will now be described by way of example only and with reference to the accompanying drawings, in which:

Figure 1 illustrates a force plate according to an embodiment of the invention;
Figure 2 illustrates a system comprising a force plate and a computer according to an embodiment of the invention;
Figure 3 shows a user standing on a force plate according to an embodiment of the invention;
Figure 4 is a flow diagram illustrating the processing performed in order to analyse the user's motion, according to an embodiment of the invention;
Figure 5 illustrates a force plate according to an embodiment of the invention;
Figure 6 illustrates the forces on an X sensor according to an embodiment of the invention;
Figure 7a is a front schematic view of a golfer in a golfing stance prior to starting a golf swing;
Figure 7b is a side schematic view of a golfer in a golfing stance prior to starting a golf swing;
Figure 8 is a schematic view of a golfer performing a golf swing;
Figure 9 illustrates the calculation of the horizontal torque component;
Figure 10 illustrates the calculation of the vertical torque component
Figure 11 is a screen capture of the welcome screen displayed by example software according to an embodiment of the invention;
Figure 12 is a screen capture of the main menu displayed by example software according to an embodiment of the invention;
Figure 13 is a screen capture of the information displayed during swing analysis by example software according to an embodiment of the invention;
Figure 14 is a screen capture of the information displayed during two swing comparison by example software according to an embodiment of the invention; and
Figure 15 is a screen capture of the swing library displayed by example software according to an embodiment of the invention.

A force plate 1 comprises a base plate 2 for the mounting of force sensors 11a, 11b, 12, 13, and a signal conditioner 14; a cover plate 3 disposed over the base plate 2; a pressure mat 4 disposed over the cover plate; and a turf mat 5 arranged on the top of the pressure mat 4.

A total of seven force sensors are mounted on the base plate 2: two X sensors 11 a and 11b arranged to measure horizontal forces in the X direction; one Y sensor 12 arranged to measure horizontal forces in the Y direction; and four Z sensors 13 one at each corner of the base plate 2 arranged to measure vertical forces. The sensors are all commercially available strain gauge load transducers. The sensors are connected to a commercially available signal conditioner 14 for strain gauge sensors which delivers an excitation voltage to the sensors, has a programmable sample rate and comprises an analog-to-digital converter. The X sensors are placed towards the front and back of the force plate, each approximately 24cm from the force plate centre in the Y direction.

The pressure mat 4 comprises a grid of force sensing resistors (FSRs). The number of FSRs can be chosen according to the desired resolution. In this embodiment, the pressure mat 4 comprises 32 rows and 64 columns of FSRs = 2048 FSRs in total, on a mat of 800mm x 500mm, resulting in a sensor spacing of approximately 14-15mm.

Two multiplexers 23A and 23B and microcontroller 24 are provided inside the force plate 1 (not shown in Figure 1). The FSRs are connected to the multiplexers 23A and 23B and the multiplexers are connected to the microcontroller 24.

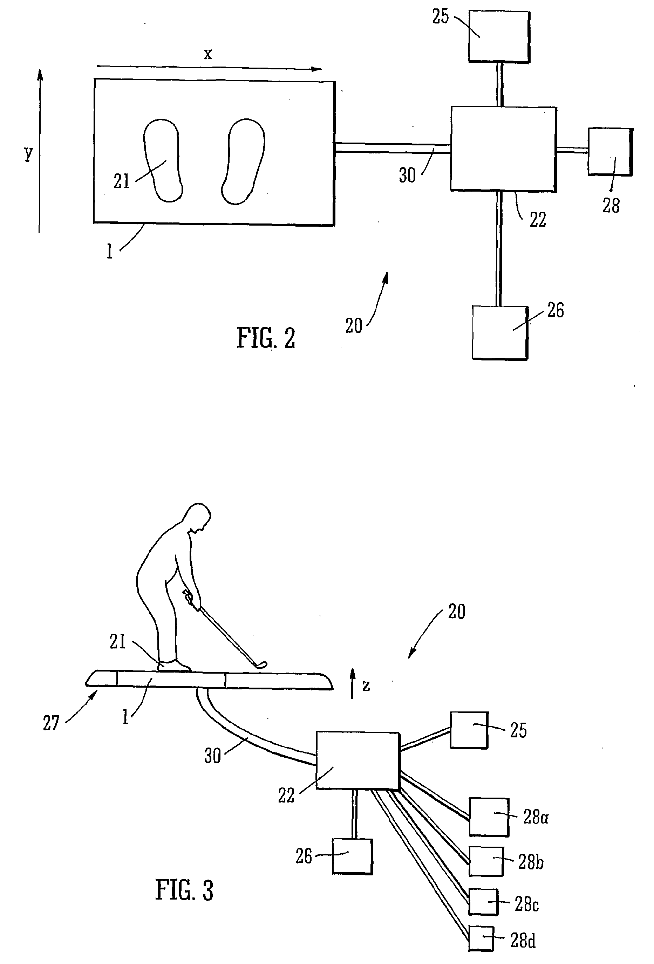

A system 20 according to an embodiment of the invention is shown in Figures 2 and 3. The force plate 1 is integrated into a tee unit 27 for teeing the ball, and is connected to a computer 22 via a USB cable 30 (in an alternative embodiment wireless communication can be used). The outputs from the signal conditioner 14 (i.e. information from the force sensors) and the microcontroller 24 (i.e. information from the pressure mat) are both input to a USB hub, which outputs the signals via USB cable 30 to the computer 22. The microcontroller controls the sampling, the multiplexer and converts signals from the pressure mat from analogue to digital.

The computer comprises software that is arranged to evaluate a variety of different types of information (denoted herein as "attributes") using these outputs, as will further be described below. The computer includes a touch-sensitive monitor (not shown) via which the information can be displayed and a person can interact with the system.

The system 20 further includes a microphone 25 and four video cameras 28A, 28B, 28C and 28D connected to the computer (only one video camera is illustrated in Figure 2 for clarity). The video cameras are positioned to collect video data of the golf swing from different viewpoints. Camera 28A is positioned along the target line, viewing the shot direction; camera 28B is positioned face on, viewing the front of the subject; camera 28C is positioned behind the subject, viewing the back of the subject; and camera 28D is mounted in the ceiling looking down on the subject. A TrackMan™ (http://trackmangolf.com/) device 26 is also connected to the computer. This evaluates the motion of the golf club and ball using doppler radar (in alternative embodiments other known motion sensors / launch monitors are used).

The system is intended to be used either by a student alone or the student and his instructor. In the former case the student will operate the system (i.e. turn it on, interact with the computer etc), the system being an independent-learning / practice tool. In the latter case it is intended that the instructor will operate the system, the system being a teaching tool to assist the instructor in coaching his student. In the embodiment described herein, the computer software has various "modes" to suit different types of practice/teaching. The term "student" used herein means the person who stands on the force plate and hits the ball.

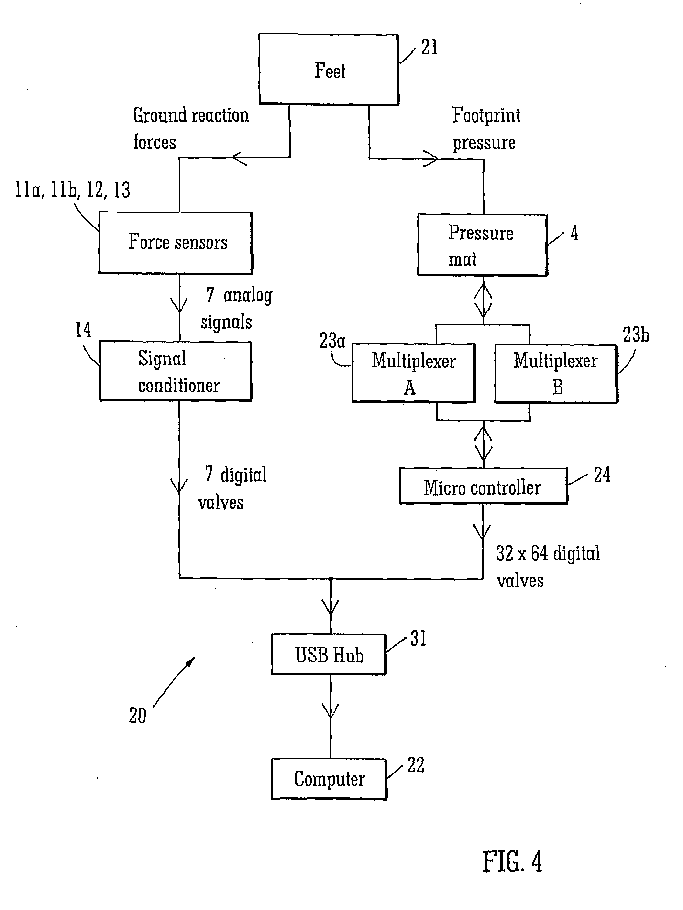

In use, a student (or his instructor) turns on the system 20 and initialises it for use using the touch-sensitive monitor. As illustrated in Figure 3, the student stands on the top of the force plate 1, i.e. on the turf mat 5. The tee unit 27 tees the ball, and the user takes a swing. The seven sensors 11a, 11*b, 12 and 13 measure the ground reaction forces of the student's feet 21 in the X, Y and vertical (Z) directions respectively and output the seven resulting analog signals to the signal conditioner 14. Figure 4 is a flow diagram illustrating the information flow. The signal conditioner 14 converts the analog signals from the sensors into digital values. These are then output to the computer 22 via the USB hub 31.

The microcontroller 24 decides which grid point to be read from the pressure mat 4 and sends a control signal to the multiplexers 23A and 23B. It also provides a 5V output to multiplexer 23A. Multiplexer 23A sequentially applies 5 volts on the 32 rows and B sequentially reads the 64 columns of the pressure mat. This enables the pressure at a single grid point to be measured at a particular time, by choosing a particular row and column.

The multiplexer 23B multiplexes the analogue grid point readings (one for each of the 2048 FSRs) into one analog signal and outputs this to the microcontroller 24. The microcontroller 24 performs analogue-digital conversion and outputs the digital pressure information, 2048 digital values, to the computer 22 via the USB hub 31. Data collected by the TrackMan™ device 26 relating to the ball and golf club motion, and video data from the video cameras 28A, B, C and D are also output to the computer.

The computer software "listens" to the output of the microphone device 25 and assesses the time of ball strike (impact) when it "hears" a noise which meets characteristics predetermined to indicate a ball strike. This is used to estimate the start and end time of the golf swing, by assuming that a golf swing should last 5 seconds in total: 3 seconds before the ball strike and 2 seconds after. These estimated times allow the sensor data to be matched with the ball strike and enable video clips to be generated that are synchronised on the ball strike.

The computer 22 stores the received data. The computer is loaded with software which is programmed to take the outputs from the signal conditioner 14 and microcontroller 24 and calculate a variety of data (attributes) related to the motion of the user. In particular, the computer estimates the position of each foot by analysing the pressure readings and determining which areas of the mat 4 are pressed down by a foot. If two islands of areas are identified each having a minimum area, they are identified as footprints (in a different embodiment the microcontroller performs this computation itself and outputs it to the computer).

Table 1 provides details of attributes calculated using the output of the pressure mat 4, whilst Table 2 provides details of attributes calculated using the output of the force sensors 11a, 11b, 12, 13, in some cases in combination with the output of the pressure mat. Conventional "phase" terminology for golf swings is used as follows:

1 st phase = address (standing still)
2nd phase = take away (move club away from ball)
3rd phase = down swing (acceleration towards ball)
4th phase = follow through (short period after impact)
5th phase = relax (deceleration of swing)
6th phase = final pose (standing still after completing a swing)

The term "Spin kick" is used to express the forces related to rotating the hip during the golf swing. This is found by estimating the torque exerted by the feet around the Z axis since this is strongly related to the force exerted by rotating the hip during a golf swing. A method of determining the spin kick magnitude is described in detail after the Tables.

Table 1

| Attribute | Measurement Unit & type | Description | Algorithm details |
|---|---|---|---|
| Left Footprint | Footprint info Continuous (footprints measured continuously during swing) | The position and outline of the left foot, relative to the top surface of the force plate | Estimate outline by measuring FSR outputs from pressure mat and determining which area of mat is pressed down by foot. If two islands of areas can be identified each having a minimum area each, they are identified as the left and right footprints. An estimation algorithm can be used to enhance accuracy by comparing a typical footprint with the measured footprint. For example some edges of the islands can be ignored if it can be identified that the foot probably does not cover the entire area of that sensor spot (which will be the case for many of the sensors along the island edges). |
| Right footprint | Footprint info Continuous | Position and outline of the right foot, relative to the force plate top surface. | As above for left footprint |
| Stance width | Centimetres Single value | How wide the feet are placed apart, measured from the centre of each footprint. | Calculate distance on the X-axis between the centre of each footprint during the address phase. |
| Stance depth | Centimetres | Deviation in position of left and right foot in the heel-toe direction. | Calculate distance on the Y-axis between the centre of each footprint during the address phase. |
| Stance angle | Degrees Single value | An angle describing how open or closed the stance is in relation to the shot direction. A stance of zero degrees is a perfectly neutral stance, with both feet parallel to the force plate. | Use trigonometry to find the angle between the line formed between the centre of each measured footprint, and the aiming line of the shot direction. The shot direction is assumed to be parallel with the tee unit, i.e. along the X axis of the force plate. |

Table 2

| Attribute | Measurement unit & type | Description | Algorithm details |
|---|---|---|---|
| Balance | % Continuous | Where the balance between the heel and the toe is during the swing. Ranges from 100% on the heel to 100% on the toe. 0% means the balance is at the centre of the foot. | Compare the centre of pressure (COP) measured by the force plate to the footprint positions found from the pressure mat, and determine where the COP lies in relation to the heel and toe. Only determine sum of balance as no need to distinguish between the feet. |
| Centre of Pressure (COP) | Position Continuous | Represents user's weight position in the horizontal plane | Calculate COP using the forces measured by the four Z sensors and the dimensions of the force plate. Calculate in standard way using linear algebra. |

(continued)

| Attribute | Measurement unit & type | Description | Algorithm details |
|---|---|---|---|
| Weight distribution | % Continuous | How the weight is distributed between the left and right feet during the swing. Ranges from 100% on left foot to 100% on right foot. | Compare COP measured by the force plate to the footprint positions found from the pressure mat, and determine where the COP lies between the left and right feet. |
| Rotational strength | Newton Single value per time interval | The sum of all rotational forces during the golf swing for a particular rotational motion, e.g. spin kick. This sum represents how much power the user generates in the rotational movement. | Sum measurements for a continuous rotational attribute such as spin kick or total body rotation for the duration of the interval. |
| Forward/ backwards shift | Newton Single value per time interval | The sum of forces exerted by shifting the feet forwards or backwards during the golf swing | Sum absolute value (so negative values contribute positively) of forces exerted on the Y sensor for the duration of the interval. |
| Sideways shift | Newton Single value per time interval | The sum of forces exerted by shifting the feet sideways during the golf swing. | Sum absolute value (so negative values contribute positively) of forces exerted on the X sensors for the duration of the interval. |
| Overall smoothness | % Single value per time interval.<br><br>Higher % = better smoothness | The degree of smoothness in the golf swing motion.<br>A rushed back swing or a swing plane that demands a lot of compensation with hand-power will produce a more jerky motion, and this will be reflected in the overall smoothness attribute. | All continuous motion attributes v. time such as COP, spin kick, shifts and rotational forces can be used as a base for evaluating the smoothness of the golf swing.<br>By differentiating the chosen data with respect to time in the 2nd and 3rd order, the speed and acceleration of the rate of change can be found at a given time.<br>A number of methods can be used to measure how smooth the change in data is. One method is to find the average absolute acceleration for the time interval and rate a lower average with a higher percentage. |
| Balance at end of swing | % Single value | Degree of balance maintained at the end of the swing. The lower the %, the higher the rate and magnitude of variation in movement, i.e. if losing balance or ending swing with sudden jerk. | Evaluate stability of COP for 5th phase and express as % |
| Rhythm | Fraction Single value | Compares the duration of the takeaway and the down swing | Calculate rhythm R as R = (Tta/Tds) Tta = duration of phase 2 (takeaway). Identify start of swing motion and top of back swing by evaluating motion |
| | | | characteristics. Tds = duration of phase 3 (down swing). Identify top of back swing as above and time of impact using microphone. |

(continued)

| Attribute | Measurement unit & type | Description | Algorithm details |
|---|---|---|---|
| Vertical swing power | Newton Single value | Difference between the maximum and minimum vertical force. The min force is generated during the down swing and the max force is generated just before impact. | Swing strength = VFmax - VFmin Where VF is the net sum of the four Z sensors at a particular time after substracting the person's weight |
| Tempo | milliseconds Single value | Time from the start of the takeaway to the impact | Tempo = $(T_{imp} - T_{start})$ $T_{imp}$ = time of impact (via microphone) $T_{start}$ = time of takeaway (find by analysing force plate data to identify when the user is moving according to the start of a golf swing. |
| Torque around Y and X axes, | Newton metre Continuous | Torque generated by body around Y and X axes, caused by leaning in a direction during the golf swing | Described below |
| Angular momentum | Newton metre seconds Single value per time interval | Angular momentum generated by body around Y & X axes, caused by leaning in a direction for the duration of the time interval. | Described below Angular momentum is torque integrated over a time interval. |
| Spin kick turn timing | milliseconds Single value | Timing of the spin kick turning point (i.e. where user shifts from accelerating to decelerating) in relation to impact | Timing = $(T_{imp}-T_{sk})$ $T_{sk}$ = time of spin kick turning point (found by analysing sensor data) |
| Maximum spin kick magnitude | Newton Single value | The maximum measured spin kick during the swing | Find maximum peak of spin kick |
| Maximum spin kick timing | milliseconds Single value | Timing of maximum spin kick (i.e. maximum spin kick acceleration towards the ball) in relation to impact | Timing = $(T_{imp}- T_{skm})$ $T_{skm}$ = time of max spin kick acceleration in relation to ball impact (found by analysing sensor data) |
| Minimum spin kick magnitude and timing | As for maximum | As for maximum This shows when maximum deceleration of hip rotation happens and how great the deceleration is. | As for maximum |
| Top pause | milliseconds Single value | The duration of the turning point between the back swing and the start of the down swing | Find negative peak of total vertical force at the transition between take away and down swing phases. Top pause is the duration of the approximately flat part of the peak. |
| Weight of user | Kg Single value | The measured weight of the user | Average total vertical force over time / gravitational acceleration |

Where the measurement is given as a percentage (except for balance and weight distribution), 100% means the measurement is perfectly close to a pre-defined ideal, whilst 0% is outside a pre-defined reasonable limit. Thus the higher the percentage, the better that aspect of the swing.

A method of determining the "spin kick" according to a preferred embodiment of the invention is now described in relation to Figures 5 and 6. Figure 5 illustrates the feet on the force plate 1. The torque exerted by the feet around the Z axis (in the XY plane) is:

$$\text{Torque} = \text{rotational force F x distance to centre of rotation D}$$

The centre of rotation CoR is assumed to be the midpoint between the feet, which can be determined in a straightforward manner from the foot positions found using the pressure mat 4. As the position (e.g. the x and y coordinates) of the X sensors 11a and 11b are known, the distances Dx and Dy in the x and y directions respectively from each X sensor to the centre of rotation (i.e. the x and y components of D) can be calculated. These distances are illustrated in Figure 5 for sensor 11 a. The direct distance D from X sensor 11 a to the centre of rotation CoR can then be found for example by using Pythagoras:

$$D = \sqrt{(Dx^2 + Dy^2)}$$

The rotational force F is then found. The approximate angle at which the force passes through the sensor is φ1 as can be seen in Figures 5 and 6. φ2 is geometrically related to φ2, since the force vector F is perpendicular to distance D. Therefore:

$$\varphi 1 = 90° - \varphi 2$$

where: tan φ2 = Dy / Dx
thus: φ2 = tan$^{-1}$ (Dy / Dx) Dy and Dx known see above

It can be seen from Figure 6 that:

$$F \approx Fx_a / \cos \varphi 1$$

Therefore

$$F \approx Fx_a / \cos (90° - (\tan^{-1} (Dy / Dx)))$$

Where: Dy and Dx are known see above
Fx$_a$ is the force measured by the X sensor 11 a

Thus the torque measured using X sensor 11 a is:

$$\text{Torque} = Fx_a / \cos (90° - (\tan^{-1} (Dy / Dx))) x \; \sqrt{(Dx^2 + Dy^2)}$$

Corresponding calculations are then performed to find the torque for the other X sensor 11b, and the two values are summed. The "spin kick" is considered to be this total torque exerted on the X sensors. The spin kick will vary over the course of the swing and thus it is measured at various time intervals and a curve of spin kick v. time is obtained.

A method of determining the torque / angular momentum around the Y and X axes (in the XZ and YZ planes) according to a preferred embodiment of the invention is now described in relation to Figures 7 to 10.

The angular momentum of a subject can be approximated as the motion of leaning in a direction while standing stationary with the feet. Consider the person as a sphere with a diameter equal to the person's height. The angular momentum is the pace at which the sphere "rolls" when leaning in a direction. Angular momentum around the Y axis is caused by leaning left or right, while in the X axis it is caused by leaning forwards or backwards. Figures 7a and 7b illustrate a golfer in this co-ordinate system preparing to take a swing, whilst Figure 8 illustrates the golfer during the swing in relation to the notional sphere.

A method for calculating the angular momentum around the Y and X axes will now be described. Let:

$Fz_1(t)$, $Fz_2(t)$, $Fz_3(t)$, $Fz_4(t)$ = the four Z sensors (measuring vertical forces), placed towards each corner of the rectangular force plate

Fz(t) = sum of forces measured by all four Z sensors at time t

Fx(t) = sum of forces measured by the two X-sensors at time t (note the X sensors are mounted so that they measure positively when clockwise torque is applied to the plate. This means X sensor 11 a is positive in the positive X axis, and 11b is negative in the positive X axis).

Fy(t) = force measured by the single horizontal Y-sensor at time t. (note that the sensors are arranged to absorb the entire force, so the sum of the forces is the total force exerted. If more sensors were used, each sensor would absorb less force and so the sum would be substantially the same).

COM(t) = Centre of Mass, the centre point of the body's mass in space. Typically this is at the centre of a person. When a person is standing in a golfing stance, the centre of the person is approximately at half the person's height, in the region of the person's bellybutton. Calculation of the COM is described later. The COM has x, y and z components.

COP(t) = Centre of Pressure, the point on the force plate that constitutes the centre of the vertical forces. When standing still, this is typically at the midpoint between the feet, see Figures 7a and 7b. Calculation of the COP is described later.

$Fz_{NET}$ (t) = net vertical force, when ignoring the constant force of gravity caused by the weight of the person, i.e. $Fz_1(t) + Fz_2(t) + Fz_3(t) + Fz_4(t) - F_{WEIGHT}$

$F_{WEIGHT}$ = vertical force caused by gravity and the weight of the person, i.e. $Fz_1(t0) + Fz_2(t0) + Fz_3(t0) + Fz_4(t0)$ when the person is standing still.

$$\text{Weight (Kg)} = F_{WEIGHT} / g \quad [\, g = \text{acceleration due to gravity, approx. } 9.81 \text{ms}^{-2}]$$

It is well known that:

$$\text{Torque} = \text{rotational force F} \times \text{perpendicular distance from the pivot to the line of action of the force (torque arm)}$$

To find the torque around the Y axis, the X (horizontal) and Z (vertical) torque components need to be found. Let:

$$\text{X torque component} = \tau_x\,(t)$$

$$\text{Z torque component} = \tau_z\,(t)$$

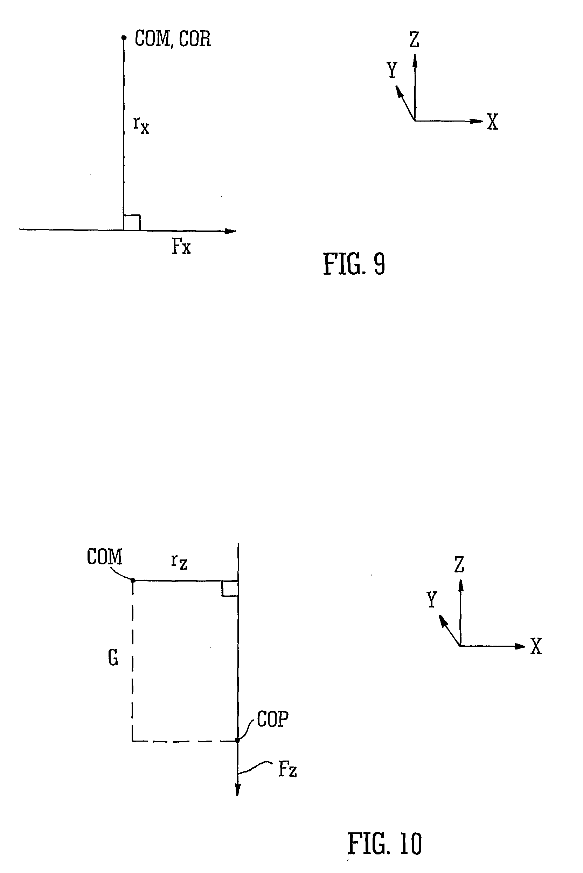

Considering first the horizontal torque component, this is generated by exerting horizontal forces Fx with the feet which are measured by the X sensors. The centre of rotation COR can be approximated as the centre of mass COM as illustrated in Figure 9. The perpendicular distance $r_x$ from the pivot to the line of action of the force Fx is therefore the vertical distance from the COM to the plane on which the X sensors are positioned, $r_x$, (sometimes denoted the "torque arm").
Thus:

$$\tau_x\,(t) = Fx(t) \times COM_z(t)$$

where: $COM_z(t)$ = position of COM in Z direction
Regarding the vertical torque component, this is generated due to the force of gravity when the person leans to one side, resulting in a deviation between the COM and COP. As above, the centre of rotation can be assumed as being the COM of the person at half the person's height. When standing still (in other words when the mechanical system is in full static equilibrium), the horizontal position of COM and COP is approximately equal, as can be seen in Figures 7a and 7b. However when the person starts moving around (while the feet remain stationary, such as in a golf swing) as shown in Figure 8 the COM and COP move, in particular the COP (i.e. the centre of the vertical forces measured by the Z sensors) moves to one side. As can be seen from Figure 10, the perpendicular distance $r_z$ (vertical torque arm) from the COM to the line of action of the vertical forces is equal to this horizontal deviation of the COP from the COM. Thus:

$$\tau_z(t) = Fz(t) \times [COP_x(t) - COM_x(t)]$$

where: $COP_x(t)$ = position of COP along X axis
$COM_x(t)$ = position of COM along X axis

Together, these two constitute the total torque exerted by the body seen as a sphere. Thus, the torque $\tau_{xz}(t)$ around the Y axis (in the XZ plane) is:

$$\tau_{xz}(t) = \tau_x(t) + \tau_z(t)$$

and therefore:

$$\tau_{xz}(t) = Fz(t) \times [COP_x(t) - COM_x(t)] + Fx(t) \times COM_z(t)$$

The COM is estimated during the golf swing as follows. Apply a double integral in the time domain of the acceleration exerted onto the body in all three axes, from the start of the golf swing up until time = t. Assume that the person stands approximately still at the start of the golf swing, at time t = t0. The COM at t=t0 must be added to the double integral. At t=t0 it can be assumed that COMx and COMy = 0. COMz can be assumed to be H/2, approximately half the person's height H (see Figure 7a).

$$COM_x(t) = \int_{t0}^{t}\int_{t0}^{t} AccelX(t)\, dt\, dt = \int_{t0}^{t}\int_{t0}^{t} \frac{Fx(t)}{Weight}\, dt\, dt$$

$$COM_y(t) = \int_{t0}^{t}\int_{t0}^{t} AccelY(t)\, dt\, dt = \int_{t0}^{t}\int_{0}^{t} \frac{Fy(t)}{Weight}\, dt\, dt$$

$$COM_z(t) = \int_{t0}^{t}\int_{t0}^{t} NetAccelZ(t)\, dt\, dt + COM_z(0) = \int_{t0}^{t}\int_{t0}^{t} \frac{Fz_{net}(t)}{Weight}\, dt\, dt + \frac{H}{2}$$

Where: Fz = vertical force measured by the z vertical force sensors (result of body weight + body motion against the floor)

$Fz_{net}$ = net vertical force, where force of gravity caused by mass of body and acceleration due to gravity (mg) is subtracted from Fz (result of body motion against the floor).

When calculating the Z-component of COM, subtract the negative normal force from the ground which is a result of the positive force of gravity and the person's weight. This constant force should be included in the equation to obtain the net vertical force, and thus the net acceleration of the body mass. Then:

$$\overrightarrow{COM}(t) = \left[COM_x(t), COM_y(t), COM_z(t)\right]$$

The Centre of Pressure is the actual measured centre point of the four vertical force sensors. Because the dimensions of the force plate and the positions of the vertical sensors are known, the position of the COP can be derived by comparing each sensor position vector $\vec{r}$ with the respective vertical force measured at the sensor at time = t, and finding the average position vector.

$$\overrightarrow{COP}(t) = \frac{\sum_{i=1}^{4} Fz_i(t) \times \vec{ri}}{Fz(t)}$$

The angular momentum around the Y and X axes respectively is found by integrating the torque over time for the respective axes.

Thus, the angular momentum $L_{XZ}(t)$ around the Y axis (in XZ plane) can be found according to the following equation:

$$L_{xz}(t) = I \times \omega_{xz} = \int_{t0}^{t} \tau_{xz}(t)\ dt$$

$$= \int_{t0}^{t} \left(Fz(t) \times (COP_x(t) - COM_x(t)) + Fx(t) \times COM_z(t)\right) dt$$

$$= \int_{t0}^{t} \left(Fz(t) \times \left(\frac{\sum_{i=1}^{4} Fz_i(t) \times \vec{ri_x}}{Fz(t)} - \int_{t0}^{t}\int_{t0}^{t} \frac{Fx(t)}{Weight}\ dt\ dt\right) + Fx(t) \times \int_{t0}^{t}\int_{t0}^{t} \frac{Fz_{net}(t)}{Weight}\ dt\ dt + \frac{H}{2}\right)\ dt$$

Considering now the X axis (YZ plane), all references to the X horizontal direction in the above equations can simply be replaced with the Y direction. Thus:

Angular momentum L around the X axis:

$$L_{yz}(t) = I \times \omega_{yz} = \int_{t0}^{t} \tau_{yz}(t)\ dt$$

$$= \int_{t0}^{t} \left(Fz(t) \times \left(COP_y(t) - COM_y(t)\right) + Fy(t) \times COM_z(t)\right) dt$$

$$= \int_{t0}^{t} \left(Fz(t) \times \left(\frac{\sum_{i=1}^{4} Fz_i(t) \times \vec{ri_y}}{Fz(t)} - \int_{0}^{t}\int_{t0}^{t} \frac{Fy(t)}{Weight}\ dt\ dt\right) + Fy(t) \times \int_{t0}^{t}\int_{t0}^{t} \frac{Fz_{net}(t)}{Weight}\ dt\ dt + \frac{H}{2}\right)\ dt$$

The above described spin kick and torque/angular momentum around the Y and X axes are particularly useful attributes for analysing golf swing motion.

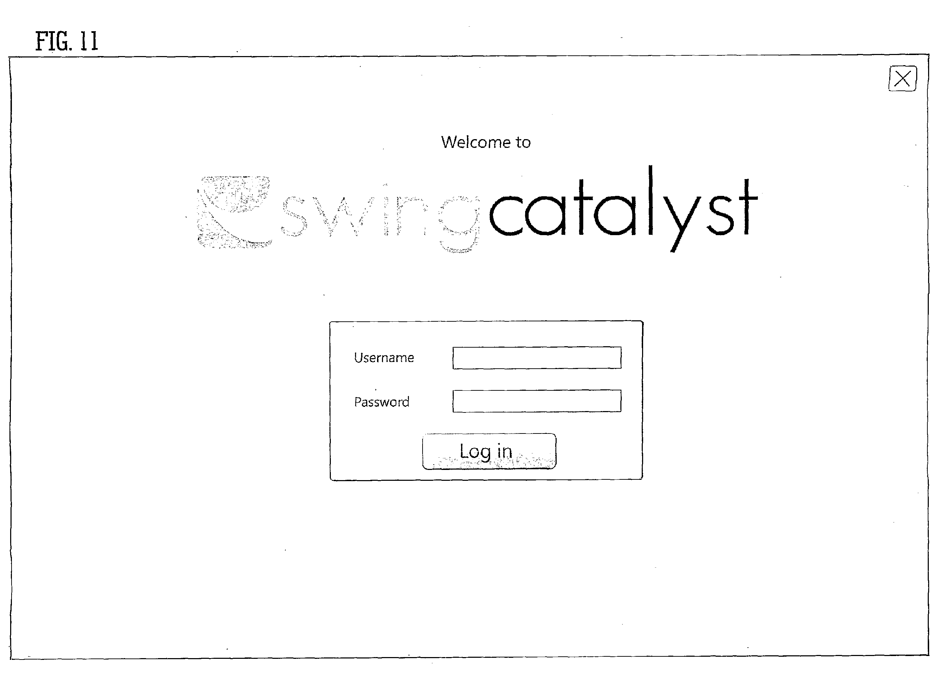

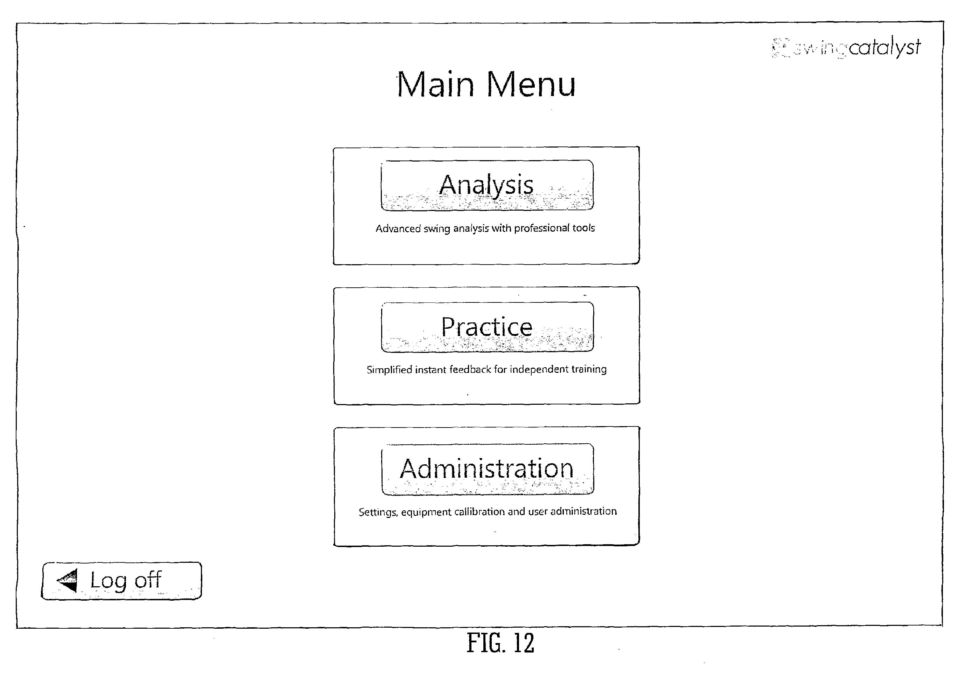

The computer 22 further comprises software that enables the student and/or his instructor to interact with the system and which displays various selected attributes on the monitor in a user-friendly manner to enable the student and/or his instructor to review performance. Figure 11 is a screen capture of the welcome screen according to an embodiment of the system of the invention. In this embodiment the system is named "swing catalyst". The student/instructor inputs a username and password in order to gain access to the system. They are then presented with the Main Menu as shown in Figure 12. This embodiment has two different "modes" of operation: Analysis mode, wherein attributes are displayed in an advanced way, more suitable for use when an instructor is present; and Practice mode which provides simplified feedback, more suitable for use by a student alone.

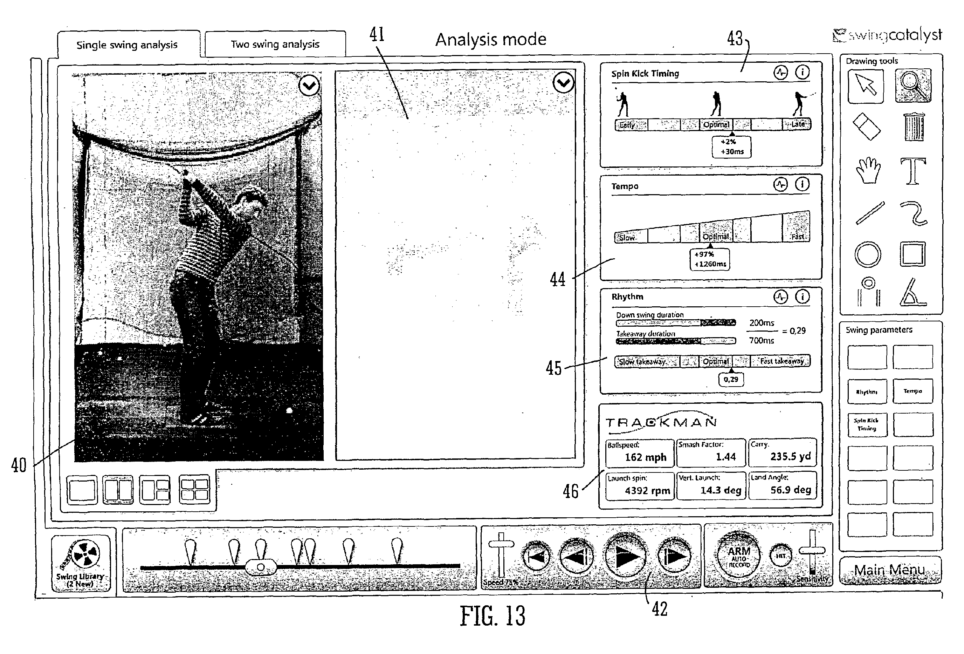

Figure 13 is a screen capture of the system in a single swing analysis mode. A video clip 40 captured by video camera 28A and a video clip 41 (image not shown) captured by video camera 28B are shown, showing the swing from different angles. This video can be played back using the controls 42. Measured data of the spin kick timing, temp and rhythm is illustrated in boxes 43, 44 and 45, and is shown as compared to the optimal performance to enable quantified evaluation of the swing. Data collected by the TrackMan™ 27 is displayed in box 46.

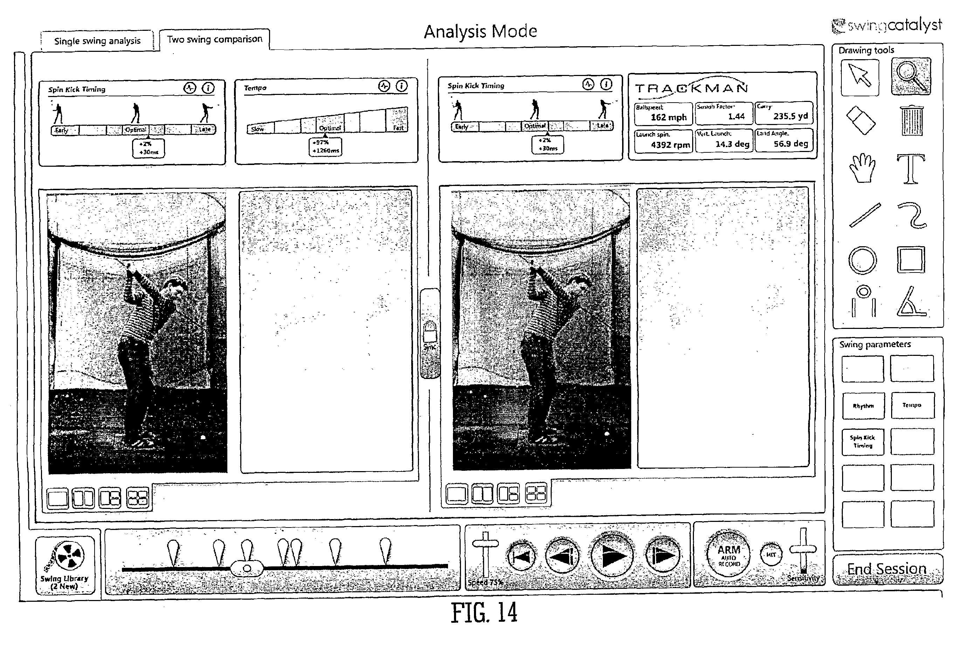

Figure 14 is a screen capture of the system in two swing comparison analysis mode. As can be seen, data relating to two swings is shown to enable comparison between the two swings.

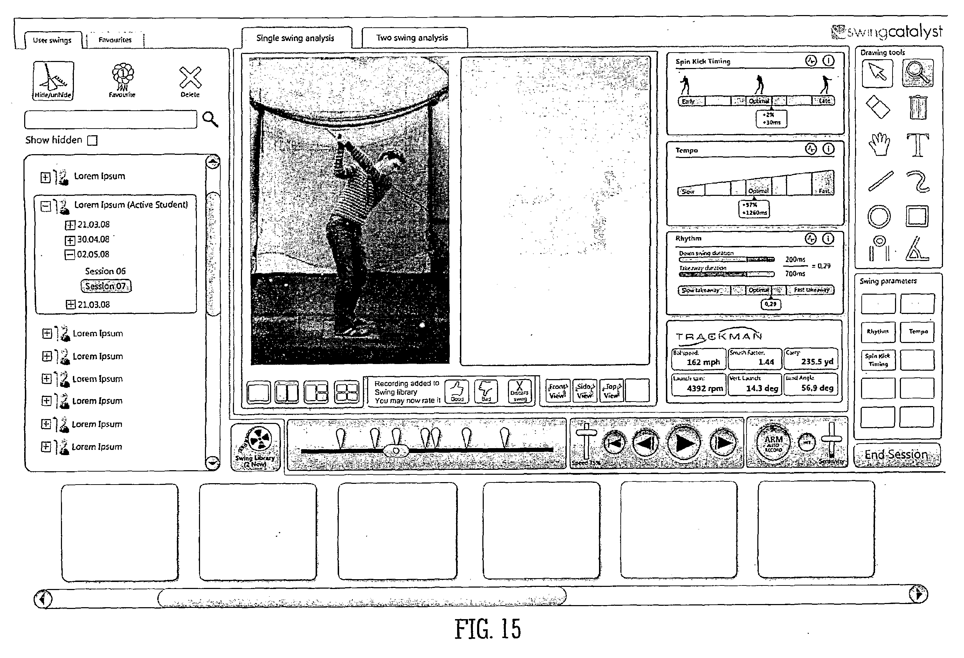

A screen capture of a swing "library" is shown in Figure 15. This enables the student/instructor to navigate through previously recorded swings using the file structure on the left. The data displayed on the right is the data for the selected recorded swing, in this example the swing of Figure 3.

As discussed above, the illustrated system is arranged to analyse motion during a golf swing and thus the computer is programmed to calculate attributes relevant to a golf swing, such as those listed in Tables 1 and 2. Of course many of these attributes (and the footprint data) would be relevant in other uses such as analysis of a baseball swing, although further attributes may also be desirable.

**Claims**

1. A sports motion analysis apparatus for use in analysing the motion of a human subject in sports, comprising a single platform (1) upon which the subject stands, the platform comprising force sensors (11a, 11b, 12, 13) arranged to measure force exerted on the platform by the subject in each of three dimensions;
   the apparatus further comprising:

   a foot position data collector (4) arranged to collect data relating to the position of each foot (21) of the subject on the platform; and
   an output arranged to output the force data and data relating to the position of each foot.

2. An apparatus as claimed in claim 1, wherein two X force sensors (11a, 11b) are provided, aligned with a X axis, for measuring force in a horizontal X direction; wherein preferably one Y force sensor (12) is provided, aligned with a Y axis, for measuring force in a horizontal Y direction which is perpendicular to the X direction; and/or wherein four Z force sensors (13) are provided, aligned with a Z axis, for measuring force in a vertical Z direction.

3. An apparatus as claimed in claim 1 or 2, wherein the force sensors (11a, 11b, 12, 13) are strain gauge load transducers; and/or wherein the foot position data collector comprises a pressure sensitive mat (4), the pressure sensitive mat preferably comprising a grid of force sensitive resistors (FSRs).

4. An apparatus as claimed in any preceding claim, further comprising electronics (14, 23a, 23b, 24, 31) arranged to control the force sensors (11a, 11b, 12, 13) and foot position data collector (4), collect data from them and output this data; and/or wherein the apparatus is arranged to output the measured data to a processor (22) which is arranged to process the data and calculate information relating to the motion of the subject.

5. A sports motion analysis system (20) for analysing the motion of a human subject in sports, comprising an apparatus according to any preceding claim; a processor (22) arranged to determine information relating to the motion of the subject using the measured force in each of three dimensions and/or data relating to the position of each foot (21); and an output to output the motion information.

6. A system as claimed in claim 5, wherein the foot position data collector comprises a pressure sensitive mat (4); and wherein the processor (22) is arranged to determine the pressure distribution of each foot from the pressure sensitive mat data; and/or to determine the outline of each foot on the platform from the pressure sensitive mat data and to use this together with the measured force in three dimensions to calculate motion information of the subject.

7. A system as claimed in claim 5 or 6, wherein the motion information is evaluated using both the measured force in each of three dimensions and data relating to the position of each foot, and wherein the motion information comprises rotation of the subject around X and/or Y and/or Z axes; and/or wherein the system further comprises a monitor for displaying the motion information; and/or wherein the system further comprises at least one video camera (28) arranged to capture video of the motion of the subject.

8. A golf practice system comprising a system as claimed in any of claims 5 to 7, further comprising one or more of the following components:

a sensor (26) arranged to measure aspects of motion of a golf club;
a sensor (26) arranged to measure aspects of the motion of a ball;
a microphone (25) for collecting sound data usable for determining particular events during a golf swing; and
a tee unit (27) into which the platform is integrated.

9. A sports motion analysis system (20) for analysing the motion of a human subject in sports, comprising a single platform (1) upon which the subject stands, the platform comprising force sensors (11a, 11b, 12, 13) arranged to measure force exerted by the subject in each of three dimensions; the system further comprising a foot position data collector (4) arranged to collect data relating to the position of each foot (21) of the subject; and a processor (22) arranged to determine information relating to the motion of the subject using measured forces and position data of each foot.

10. A golf practice system (20) comprising a system as claimed in claim 9, wherein the platform (1) is one on which the subject stands whilst practising a golf swing, wherein the processor (22) is arranged to receive the force data and data relating to the position of each foot (21), determine the position of each foot of the subject and evaluate motion characteristics of the subject from the force data and foot position data; the system further comprising a display arranged to display the calculated motion characteristics; wherein preferably the platform is integrated into a tee unit (27).

11. A method of analysing the motion of a human subject in sports, comprising:

measuring force exerted on a single platform (1) in each of three dimensions by a subject standing on the platform;
collecting data relating to the position of each foot (21) of the subject on the platform; and
outputting the force measurements and data relating to the position of each foot.

12. A method of analysing the motion of a human subject in sports, comprising:

measuring force exerted on a single platform (1) in each of three dimensions by the subject standing on the platform;
collecting data relating to the position of each foot (21) of the subject on the platform;
determining information relating to the motion of the subject using both the measured forces and data relating to the position of each foot; and
outputting the motion information.

13. A method as claimed in claim 11 or 12, wherein determining the motion information comprises determining torque and/or angular momentum of the subject around X, and/or Y, and/or Z axes using both the measured force in each of three dimensions and data relating to the position of each foot;
and/or wherein the data relating to the position of each foot of the subject is collected using a pressure sensitive mat (4), wherein preferably the method comprises determining the outline of the foot and/or pressure distribution of the foot from the pressure sensitive mat.

**Patentansprüche**

1. Ein Sportbewegungsanalysegerät für die Verwendung beim Analysieren der Bewegung von einem menschlichen Subjekt beim Sport, aufweisend einen einzelnen Unterbau (1), auf dem das menschliche Subjekt steht, der Unterbau weist Kraftsensoren (11a, 11 b, 12, 13) auf, die angeordnet sind, um eine Kraft zu messen, die auf den Unterbau durch das Subjekt in jede von drei Dimensionen ausgeübt wird;
die Vorrichtung weiter aufweisend:

einen Fußpositionsdatensammler (4), der angeordnet ist, um Daten mit Bezug auf die Position von jedem Fuß (21) von dem Subjekt auf dem Unterbau zu sammeln; und
eine Ausgabe, die angeordnet ist, um die Kraftdaten und die Daten mit Bezug auf die Position von jedem Fuß auszugeben.

**2.** Eine Vorrichtung wie in Anspruch 1 beansprucht, wobei zwei X-Kraftsensoren (11 a, 11 b) bereitgestellt sind, ausgerichtet auf eine X-Achse, zum Messen einer Kraft in einer horizontalen X-Richtung; wobei vorzugsweise ein Y-Kraftsensor (12) bereitgestellt ist, ausgerichtet auf eine Y-Achse, zum Messen einer Kraft in einer horizontalen Y-Richtung, die senkrecht zu der X-Richtung verläuft; und/oder wobei vier Z-Kraftsensoren (13) bereitgestellt sind, ausgerichtet auf eine Z-Achse, zum Messen einer Kraft in eine vertikale Z-Richtung.

**3.** Eine Vorrichtung wie in Anspruch 1 oder 2 beansprucht, wobei die Kraftsensoren (11a, 11 b, 12, 13) Dehnungsmessstreifen-Belastungsaufnehmer sind; und/oder wobei der Fußpositionsdatensammler eine druckempfindliche Matte (4) aufweist, die druckempfindliche Matte weist vorzugsweise ein Raster von druckempfindlichen Widerständen (FSRs) auf.

**4.** Eine Vorrichtung wie in einem vorangegangenen Anspruch beansprucht, weiter aufweisend Elektronik (14, 23a, 23b, 24, 31), die angeordnet ist, um die Kraftsensoren (11a, 11 b, 12, 13) und den Fußpositionsdatensammler (4) zu steuern, Daten von ihnen zu sammeln und diese Daten auszugeben; und/oder wobei die Vorrichtung angeordnet ist, um die gemessenen Daten an einen Prozessor (22) auszugeben, der angeordnet ist, um die Daten zu verarbeiten und Information mit Bezug auf die Bewegung von dem Subjekt zu berechnen.

**5.** Ein Sportbewegungsanalysesystem (20) zum Analysieren der Bewegung von einem menschlichen Subjekt beim Sport, aufweisend eine Vorrichtung gemäß einem vorangegangenen Anspruch; einen Prozessor (22), der angeordnet ist, um Information mit Bezug auf die Bewegung von dem Subjekt zu bestimmen, unter Verwendung der gemessenen Kraft in jeder von drei Dimensionen und/oder Daten mit Bezug auf die Position von jedem Fuß (21); und eine Ausgabe, um die Bewegungsinformation auszugeben.

**6.** Ein System wie in Anspruch 5 beansprucht, wobei der Fußpositionsdatensammler eine druckempfindliche Matte (4) aufweist; und wobei der Prozessor (22) angeordnet ist, um die Druckverteilung von jedem Fuß aus den Daten der druckempfindlichen Matte zu bestimmen; und/oder um den Umriss von jedem Fuß auf dem Unterbau aus den Daten der druckempfindlichen Matte zu bestimmen und um dies zusammen mit der gemessenen Kraft in drei Dimensionen zu verwenden, um Bewegungsinformation von dem Subjekt zu berechnen.

**7.** Ein System wie in Anspruch 5 oder 6 beansprucht, wobei die Bewegungsinformation evaluiert wird unter Verwendung von sowohl der gemessenen Kraft in jeder von drei Dimensionen als auch Daten mit Bezug auf die Position von jedem Fuß und wobei die Bewegungsinformation Rotation von dem Subjekt um X- und/oder Y- und/oder Z- Achsen aufweist; und/oder wobei das System weiter aufweist einen Monitor zum Anzeigen der Bewegungsinformation; und/oder wobei das System weiter aufweist zumindest eine Videokamera (28), die angeordnet ist, um ein Video von der Bewegung von dem Subjekt aufzunehmen.

**8.** Ein Golfübungssystem aufweisend ein System wie in einem von den Ansprüchen 5 bis 7 beansprucht, weiter aufweisend eines oder mehrere von den folgenden Komponenten:

    ein Sensor (26), der angeordnet ist, um Bewegungsaspekte von einem Golfschläger zu messen;
    ein Sensor (26), der angeordnet ist, um Bewegungsaspekte von einem Ball zu messen;
    ein Mikrofon (25) zum Sammeln von Geräuschdaten, die nutzbar sind zum Bestimmen von bestimmten Ereignissen während eines Golfschlags; und
    eine Abschlageinheit (27) in welche der Unterbau integriert ist.

**9.** Ein Sportbewegungsanalysesystem (20) zum Analysieren der Bewegung von einem menschlichen Subjekt beim Sport, aufweisend einen einzelnen Unterbau (1) auf dem das Subjekt steht, der Unterbau weist Kraftsensoren (11a, 11 b, 12, 13) auf, die angeordnet sind, um eine Kraft zu messen, die durch das Subjekt in jede von drei Dimensionen ausgeübt wird; das System weist weiter einen Fußpositionsdatensammler (4) auf, der angeordnet ist, um Daten mit Bezug auf die Position von jedem Fuß (21) von dem Subjekt zu sammeln; und einen Prozessor (22), der angeordnet ist, um Information mit Bezug auf die Bewegung von dem Subjekt zu bestimmen, unter Verwendung von gemessenen Kräften und von Positionsdaten von jedem Fuß.

**10.** Ein Golfübungssystem (20) aufweisend ein System wie in Anspruch 9 beansprucht, wobei der Unterbau (1) einer ist, auf dem das Subjekt während dem Üben eines Golfschlags steht, wobei der Prozessor (22) angeordnet ist, um die Kraftdaten und Daten mit Bezug auf die Position von jedem Fuß (21) zu empfangen, die Position von jedem Fuß von dem Subjekt zu bestimmen und Bewegungscharakteristiken von dem Subjekt aus den Kraftdaten und Fußpositionsdaten zu evaluieren; das System weist weiter eine Anzeige auf, die angeordnet ist, um die berechneten

EP 2 362 803 B1

Bewegungscharakteristiken anzuzeigen; wobei der Unterbau vorzugsweise in eine Abschlageinheit (27) integriert ist.

11. Ein Verfahren zum Analysieren der Bewegung von einem menschlichen Subjekt beim Sport, aufweisend:

Messen einer Kraft, die auf einen einzelnen Unterbau (1) in jede von drei Dimensionen durch ein Subjekt, das auf dem Unterbau steht, ausgeübt wird;
Sammeln von Daten mit Bezug auf die Position von jedem Fuß (21) von dem Subjekt auf dem Unterbau; und
Ausgeben der Kraftmessungen und der Daten mit Bezug auf die Position von jedem Fuß.

12. Ein Verfahren zum Analysieren der Bewegung von einem menschlichen Subjekt beim Sport, aufweisend:

Messen einer Kraft, die auf einen einzelnen Unterbau (1) in jede von drei Dimensionen durch ein Subjekt, das auf dem Unterbau steht, ausgeübt wird;
Sammeln von Daten mit Bezug auf die Position von jedem Fuß (21) von dem Subjekt auf dem Unterbau;
Bestimmen von Information mit Bezug auf die Bewegung von dem Subjekt unter Verwendung von sowohl den gemessenen Kräften als auch den Daten mit Bezug auf die Position von jedem Fuß; und
Ausgeben der Bewegungsinformation.

13. Ein Verfahren wie in Anspruch 11 oder 12 beansprucht, wobei das Bestimmen der Bewegungsinformation aufweist Bestimmen eines Drehmoments und/oder Drehimpulses von dem Subjekt um X und/oder Y und/oder Z Achsen unter Verwendung von sowohl der gemessenen Kraft in jede von drei Dimensionen und der Daten mit Bezug auf die Position von jedem Fuß;
und/oder wobei die Daten mit Bezug auf die Position von jedem Fuß von dem Subjekt unter Verwendung einer druckempfindlichen Matte (4) gesammelt werden, wobei das Verfahren vorzugsweise aufweist Bestimmen des Umrisses von dem Fuß und/oder der Druckverteilung von dem Fuß aus der druckempfindlichen Matte.

Revendications

1. Appareil d'analyse de mouvements sportifs pour une utilisation dans l'analyse du mouvement d'un sujet humain en sport, comprenant une plate-forme unique (1) sur laquelle le sujet est debout, la plate-forme comprenant des capteurs de force (11a, 11b, 12, 13) aménagés pour mesurer la force exercée sur la plate-forme par le sujet dans chacune des trois dimensions ;
l'appareil comprenant en outre :

un collecteur de données de position de pieds (4) aménagé pour recueillir des données se rapportant à la position de chaque pied (21) du sujet sur la plate-forme ; et
une sortie aménagée pour délivrer les données de force et les données se rapportant à la position de chaque pied.

2. Appareil selon la revendication 1, dans lequel il est prévu deux capteurs de force X (11a, 11b) alignés sur un axe X pour mesurer la force dans une direction X horizontale ; dans lequel, de préférence, il est prévu un capteur de force Y (12) aligné sur un axe Y pour mesurer la force dans une direction Y horizontale qui est perpendiculaire à la direction X ; et/ou dans lequel il est prévu quatre capteurs de force Z (13) alignés sur un axe Z pour mesurer la force dans la direction Z verticale.

3. Appareil selon la revendication 1 ou la revendication 2, dans lequel les capteurs de force (11a, 11b, 12, 13) sont des transducteurs de charge à jauge de contrainte ; et/ou dans lequel le collecteur de données de position des pieds comprend une natte sensible à la pression (4), la natte sensible à la pression comprenant de préférence un réseau de résistances sensibles aux forces (FSR).

4. Appareil selon l'une quelconque des revendications précédentes, comprenant en outre des dispositifs électroniques (14, 23a, 23b, 24, 31) aménagés pour commander les capteurs de force (11a, 11b, 12, 13) et le collecteur de données de position de pieds (4), recueillir des données de ceux-ci et délivrer ces données ; et/ou dans lequel l'appareil est aménagé pour délivrer les données mesurées à un processeur (22) qui est aménagé pour traiter les données et calculer des informations concernant le mouvement du sujet.

5. Système d'analyse de mouvements sportifs (20) pour analyser le mouvement d'un sujet humain en sport, comprenant un appareil selon l'une quelconque des revendications précédentes ; un processeur (22) aménagé pour déterminer

18

des informations concernant le mouvement du sujet en utilisant la force mesurée dans chacune des trois dimensions et/ou des données se rapportant à la position de chaque pied (21) ; et une sortie pour délivrer les informations de mouvement.

6. Système selon la revendication 5, dans lequel le collecteur de données de position des pieds comprend une natte sensible à la pression (4) ; et dans lequel le processeur (22) est à même de déterminer la distribution de pression de chaque pied à partir de données de la natte sensible à la pression ; et/ou pour déterminer le profil de chaque pied sur la plate-forme à partir des données de la natte sensible à la pression et pour utiliser cette information conjointement avec la force mesurée en trois dimensions afin de calculer des informations de mouvement du sujet.

7. Système selon la revendication 5 ou la revendication 6, dans lequel les informations de mouvement sont évaluées en utilisant à la fois la force mesurée dans chacune des trois dimensions et des données se rapportant à la position de chaque pied, et dans lequel les informations de mouvement comprennent la rotation du sujet autour des axes X et/ou Y et/ou Z ; et/ou dans lequel le système comprend en outre un moniteur pour afficher les informations de mouvement ; et/ou dans lequel le système comprend en outre au moins une caméra vidéo (28) aménagée pour capter une vidéo du mouvement du sujet.

8. Système d'entraînement de golf comprenant un système selon l'une quelconque des revendications 5 à 7, comprenant en outre un ou plusieurs des composants suivants :

    un capteur (26) aménagé pour mesurer des aspects de mouvement d'un club de golf ;
    un capteur (26) aménagé pour mesurer des aspects du mouvement d'une balle ;
    un microphone (25) pour recueillir des donnée sonores utilisables pour déterminer des événements particuliers au cours d'un swing de golf ; et
    une unité de tee (27) à laquelle la plate-forme est intégrée.

9. Système d'analyse de mouvements sportifs (20) pour analyser le mouvement d'un sujet humain en sport, comprenant une plate-forme unique (1) sur laquelle le sujet est debout, la plate-forme comprenant des capteurs de force (11a, 11b, 12, 13) aménagés pour mesurer une force exercée par le sujet dans chacune des trois dimensions ; le système comprenant en outre un collecteur de données de position de pieds (4) aménagé pour recueillir des données concernant la position de chaque pied (21) du sujet ; et un processeur (22) aménagé pour déterminer des informations concernant le mouvement du sujet en utilisant les forces mesurées et les données de position de chaque pied.

10. Système d'entraînement de golf (20) comprenant un système selon la revendication 9, dans lequel la plate-forme (1) est une plate-forme sur laquelle le sujet est debout tout en pratiquant un swing de golf, dans lequel le processeur (22) est à même de recevoir les données de force et les données se rapportant à la position de chaque pied (21), déterminer la position de chaque pied du sujet et évaluer des caractéristiques de mouvement du sujet à partir des données de force et des données de position des pieds ; le système comprenant en outre un affichage aménagé pour afficher les caractéristiques de mouvement calculées ; dans lequel, de préférence, la plate-forme est intégrée à une unité de tee (27).

11. Procédé d'analyse du mouvement d'un sujet humain en sport, comprenant les étapes consistant à :

    mesurer la force exercée sur une plate-forme unique (1) dans chacun des trois dimensions par un sujet qui se trouve debout sur la plate-forme ;
    recueillir des données se rapportant à la position de chaque pied (21) du sujet sur la plate-forme ; et délivrer les mesures de force et les données se rapportant à la position de chaque pied.

12. Procédé d'analyse du mouvement d'un sujet humain en sport, comprenant les étapes consistant à :

    mesurer la force exercée sur une plate-forme unique (1) dans chacune des trois dimensions par le sujet qui est debout sur la plate-forme ;
    recueillir des données se rapportant à la position de chaque pied (21) du sujet sur la plate-forme ;
    déterminer des informations se rapportant au mouvement du sujet en utilisant à la fois les forces mesurées et les données se rapportant à la position de chaque pied ; et
    délivrer les informations de mouvement.

13. Procédé selon la revendication 11 ou la revendication 12, dans lequel la détermination des informations de mouvement comprend la détermination du couple de torsion et/ou du moment angulaire du sujet autour des axes X et/ou Y et/ou Z en utilisant à la fois la force mesurée dans chacune des trois dimensions et les données se rapportant à la position de chaque pied ; et/ou

dans lequel les données se rapportant à la position de chaque pied du sujet sont recueillies en utilisant une natte sensible à la pression (4), dans lequel, de préférence, le procédé comprend la détermination du profil du pied et/ou la distribution de pression du pied à partir de la natte sensible à la pression.

FIG. 1

EP 2 362 803 B1

FIG. 2

FIG. 3

FIG. 4

FIG. 5

EP 2 362 803 B1

FIG. 6

FIG. 7a

FIG. 7b

FIG. 8

COM, COR

$r_x$

Z
Y
X

Fx

FIG. 9

COM $r_z$

G

COP

Fz

Z
Y
X

FIG. 10

FIG. 11

Welcome to

swingcatalyst

Username [                    ]

Password [                    ]

[ Log in ]

# Main Menu

## Analysis

Advanced swing analysis with professional tools

## Practice

Simplified instant feedback for independent training

## Administration

Settings, equipment callibration and user administration

◀ Log off

FIG. 12

FIG. 13

FIG. 14

EP 2 362 803 B1

FIG. 15

EP 2 362 803 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070196800 A1

- WO 2006120658 A